# EUROPÄISCHE PATENTSCHRIFT

(11) **EP 3 487 557 B1**
(45) Veröffentlichungstag und Bekanntmachung des Hinweises auf die Patenterteilung: **03.05.2023**
(21) Anmeldenummer: 17752014.5
(22) Anmeldetag: 19.07.2017
(51) Int. Cl.: A61M 5/14, F16H 25/20, F16D 7/02

(54) **MEDIZINTECHNISCHE KLEMMVORRICHTUNG MIT SICHERHEITSKUPPLUNG**
MEDICAL-TECHNICAL CLAMP DEVICE WITH A SAFETY CLUTCH
DISPOSITIF DE SERRAGE DE LA TECHNOLOGIE MÉDICALE AVEC UN ACCOUPLEMENT DE SÉCURITÉ

(30) Priorität: 20.07.2016 DE 102016113355
(43) Veröffentlichungstag der Anmeldung: 29.05.2019
(73) Patentinhaber: B. Braun Melsungen AG, 34212 Melsungen (DE)
(72) Erfinder: ERLEN, Christoph, 34132 Kassel (DE); SPÖRL, Thomas, 34212 Melsungen (DE); WERNER, Sascha-Oliver, 37077 Göttingen (DE); SCHÜSSLER, Andreas, 34123 Kassel (DE); THOMAS, Thorsten, 34613 Schwalmstadt (DE)
(74) Vertreter: Winter, Brandl - Partnerschaft mbB
(86) Internationale Anmeldenummer: PCT/EP2017/068220
(87) Internationale Veröffentlichungsnummer: WO 2018/015427

(56) Entgegenhaltungen:
- EP-A1- 2 154 382
- WO-A1-2014/035496
- WO-A2-2005/023493
- DE-U1-202012 101 464
- JP-A- 2013 257 004
- JP-B1- 5 792 353
- US-A1- 2011 121 149
- US-A1- 2012 130 381

## Beschreibung

Die vorliegende Erfindung betrifft eine medizintechnische Klemmvorrichtung zur klemmenden Befestigung eines medizintechnischen Geräts an einer Führung, insbesondere an einer stangenartigen Führung, wobei die Klemmvorrichtung zwei zueinander mittels einer Betätigungsmechanik relativbewegbare Klemmabschnitte, insbesondere Klemmbacken, für einen klemmenden Eingriff mit der Führung aufweist.

In Krankenhäusern, Kliniken oder anderen medizinischen Behandlungsstätten werden Medizingeräte zur Durchführung von Behandlungen am Patienten direkt oder indirekt über Haltesysteme an Führungen oder Halteeinrichtungen lösbar befestigt. Solche Geräte sind insbesondere Geräte für eine Infusionstherapie, die an Stangen wie z.B. einem Infusionsständer lösbar befestigt werden. Eine weitere Möglichkeit ist eine lösbare Befestigung an Führungen oder Halteeinrichtungen, die an Krankenbetten oder OP-Tischen vorgesehen sind.

Zu diesem Zweck vorgesehene Führungen (Befestigungsstangen oder Halteeinrichtungen) sind in der Regel mit unterschiedlichen Querschnittsformen, Oberflächen und Abmessungen, insbesondere Durchmessern realisiert. Eine medizinische Klemmvorrichtung sollte an solche unterschiedliche Gegebenheiten individuell anpassbar sein und ein sicheres und einfaches Befestigen des Geräts ermöglichen. Ein dabei auftretendes Problem kann sein, dass die Oberfläche der Führungen in der Regel glatt ist, z.B. verchromt, was die Gefahr einer gewissen Gleitneigung der Klemmvorrichtung daran birgt. Dieses Problem kann dadurch verstärkt werden, dass solche Führungen regelmäßig mit unterschiedlichen Reinigungs- und Desinfektionsmitteln gereinigt und desinfiziert werden, was dazu führen kann, dass sich Oberflächeneigenschaften so verändern, dass die Gleitneigung der Klemmvorrichtung daran ansteigt. Auch können, insbesondere im Rahmen einer parenteralen Ernährung, im direkten Umfeld der Führung bzw. der Klemmvorrichtung fetthaltige Flüssigkeiten gehandhabt werden, die auf die Führungsoberfläche bzw. zwischen die Führung und die Klemmvorrichtung gelangen können, was ebenfalls zu erheblichen Veränderungen der Gleitneigung führen kann.

Bekannte Befestigungssysteme basieren auf einer an einem Medizingerät befestigten Klemmvorrichtung. In der Regel ist das Gerät mittels einer oder zweier Klemmbacken an einer Führung festklemmbar. Die Klemmbacken sind häufig über eine Spindelmechanik beweglich und zum Klemmen an der Führung zu betätigen. Es kann passieren, dass ein mittels eines solchen Befestigungssystems befestigtes Medizingerät nicht ausreichend fixiert ist und in Folge dessen abgleiten oder sich um die Führen herum verdrehen lassen kann. Dies kann beispielsweise dadurch begründet sein, dass die Führung oder die Klemmbacken wie vorstehend beschrieben verschmutzt ist bzw. sind. In der Regel versuchen Anwender im Falle einer unzureichenden Klemmung durch Erhöhen der Klemmkraft eine höhere Normalkraft auf die Klemmschiene auszuüben, um so ausreichende Haftreibung zu erzeugen. Dies kann aber in nachteiliger Weise dazu führen, dass die Klemmbacken, die Führung oder sogar die gesamte Klemmvorrichtung irreversibel zerstört werden. Schlimmstenfalls kann das Medizingerät sogar herabfallen, was je nach Anwendungsfall zu unvertretbaren Risiken führt.

Ein weiteres Problem bei bekannten medizintechnischen Klemmvorrichtungen kann durch zu starkes anwenderseitiges Aufdrehen der Spindel beim Lösen der Klemmvorrichtung entstehen. Wird diese bis zum äußeren Anschlag oder sogar darüber hinaus gelöst/ausgeschraubt, kann das je nach Ausführung dazu führen, dass sich die Spindel in dieser offenen Position in dem ihr zugehörigen Gewinde verklemmt und aus dieser Verklemmung nur schwer wieder lösbar ist. Schlimmstenfalls kann die Spindel gänzlich unlösbar sein. Folge kann wiederum eine irreversible Zerstörung einzelner Komponenten der Klemmvorrichtung oder des zu klemmenden medizinischen Geräts sein.

Ein weiteres Problem bekannter Klemmvorrichtungen kann sein, dass die Klemme nicht in der erwünschten Position an der Stange montiert wird, sondern unmerklich falsch fixiert wird. Dies kann zu einem schrägen Positionieren, Abgleiten, Wegdrehen oder zu einer Zerstörung der Klemme und/oder der Führung führen. Darüber hinaus kann es im Falle einer Führung mit Kreisquerschnitt dazu kommen, dass die Klemme mit ihrem höchsten Punkt an dem Außendurchmesser der Führung fixiert, anstatt mit einer dazu vorgesehenen Sicke oder Mulde. In einem solchen Fall ist das Medizingerät zwar temporär fixiert, allerdings nicht in einem stabilen Zustand. Wohlmöglich kann es bei sachgemäßem Manipulieren am scheinbar festgeklemmten medizintechnischen Gerät, z.B. beim Einlegen einer Spritze in ein Infusionsgerät, dazu kommen, dass die Klemmvorrichtung an der Führung abrutscht.

Beispielsweise ist aus JP 5 792353 B1 eine Klemmvorrichtung zum Befestigen eines medizintechnischen Geräts z.B. an einer Stange eines Stands bekannt, mit einem in einen Drehgriff integrierten Drehmomentbegrenzer, bei welchem Lamellenplatten durch eine Feder aneinander gedrückt und miteinander in Eingriff sind. Auch bei dieser Klemmvorrichtung treten kann es beispielsweise zu einem schrägen Positionieren, Abgleiten, Wegdrehen oder zu einer Zerstörung der Klemme und/oder der Führung kommen.

Vor diesem Hintergrund liegt der vorliegenden Erfindung die Aufgabe zugrunde, eine medizintechnische Klemmvorrichtung zu schaffen, die die vorgenannten Nachteile bekannter Klemmvorrichtungen nicht aufweist. Die Klemmvorrichtung soll nutzerfreundlich und einfach zu bedienen sein, wobei insbesondere zu starke Klemmkräfte zum Erzielen eines sicheren Halts eines zu befestigenden medizintechnischen Geräts sowie ein Festklemmen der Klemmvorrichtung im gelösten Zustand ausgeschlossen sind.

Diese Aufgabe wird nach der vorliegenden Erfindung gelöst durch eine medizintechnische Klemmvorrichtung gemäß Anspruch 1, die eine Begrenzungseinheit aufweist, die kraftabhängig, insbesondere klemmkraftabhängig beim Festklemmen der Klemmvorrichtung, und/oder wegabhängig, insbesondere wegabhängig beim Lösen der Klemmvorrichtung, zumindest einen Klemmabschnitt, insbesondere eine Klemmbacke, von der Betätigungsmechanik entkoppelt. Die Führung kann insbesondere stangenartig ausgebildet sein. Ferner weist die Betätigungseinheit eine mit einer Spindelmutter zusammenwirkende Spindel auf. Die Spindel ist vorzugsweise axialfest, aber um ihre Spindellängsachse rotierbar in der Klemmvorrichtung gelagert. Die Spindelmutter ist vorzugsweise in Richtung der Spindelachse axialverschiebbar, aber drehfest in der Klemmvorrichtung gelagert. Die Spindel kann alternativ auch axial verschiebbar und mechanisch mit der Klemmbacke nicht rotierbar verbunden sein und die Spindelmutter kann axial fixiert, jedoch über eine Drehachse rotierbar und mechanisch mit einem Drehhandgriff verbunden sein. Die Klemmvorrichtung weist ferner ein Gehäuse auf, wobei zumindest eine Außenseite des Gehäuses eine mit einer durch den Drehhandgriff positionierbaren Klemmbacke zusammenwirkende Klemmbacke ausbildet oder aufweist, und die Klemmbacken jeweils eine Anlagestruktur zum Zusammenwirken mit der Führung aufweisen.

Die Entkopplung erfolgt nach der Erfindung derart, dass eine fortgeführte Betätigung der Klemmvorrichtung nach einem Überschreiten eines definierten Grenzwerts, was die Entkopplung auslöst, nicht möglich ist, ein kurz vor der Entkopplung erreichter Zustand aber bewahrt wird. Durch die erfindungsgemäße Ausbildung der Klemmvorrichtung wird in vorteilhafter Weise bewirkt, dass bei einem Anziehen/Festziehen der Klemmvorrichtung, also bei einem Schließen der Klemmbacken zum Festklemmen an der Führung, und/oder bei einem Lösen der Klemmvorrichtung bei Erreichen eines bestimmten Grenzwerts die Betätigungsmechanik so von zumindest einer der Klemmbacken entkoppelt wird, dass fortgeführtes Betätigen über den Grenzwert hinaus nicht möglich ist oder aber ohne Wirkung ist, eine dem Grenzwert im Wesentlichen entsprechende Klemmung bzw. Relativpositionierung der Klemmbacken jedoch gehalten wird. Im Rahmen der Erfindung können der Grenzwert und damit die Entkopplung der Klemmbacke von der Betätigungseinheit kraftabhängig und/oder wegabhängig bestimmt sein und erfolgen. "Kraftabhängig" im Sinne der Erfindung beinhaltet die Bedeutungen von "betätigungskraftabhängig" wie auch von "klemmkraftabhängig". Ferner wird durch die Anordnung der Spindel und der Spindelmutter eine platzsparende Bauweise ermöglicht.

Vorteilhafte Ausführungsformen der Erfindung sind in den Unteransprüchen beansprucht und werden nachfolgend näher erläutert.

Eine Ausführungsform der Erfindung ist dadurch gekennzeichnet, dass die Begrenzungseinheit in Form einer Rutschkupplung ausgebildet ist. Diese kann unidirektional, also nur in eine Richtung (Lösen oder Klemmen) bei Überschreiten des Grenzwerts rutschen, oder bidirektional, also in beide Richtungen (Lösen und Klemmen) bei Überschreiten des Grenzwerts rutschen, wirken.

Vorzugsweise weist die Klemmvorrichtung einen Drehbetätigungsgriff auf, in den die Begrenzungseinheit integriert ist. Ein solcher Drehbetätigungsgriff kann nach der Erfindung aus zwei oder mehreren Einzelteilen zusammengefügt sein, die ein Griffgehäuse ausbilden. Die Begrenzungseinheit kann darin drehmomentbegrenzend angeordnet sein. Sie kann mechanisch über ein zwischen dem Griffgehäuse und der Betätigungsvorrichtung wirkendes Kupplungselement realisiert sein. Das Gehäuse des Betätigungsgriffs kann nach der Erfindung aus einem metallischen Werkstoff, insbesondere aus Federstahl, und/oder aus einem Kunststoff, insbesondere aus PP, PE-HD, PE-LD, POM, ABS oder einem ähnlichen technischen Kunststoff oder Kombinationen davon bestehen.

Eine weitere Ausführungsform der Erfindung ist dadurch gekennzeichnet, dass die Spindel mit dem Drehhandgriff wirkverbunden und antreibbar ist. Diese Wirkverbindung kann nach der Erfindung in vorteilhafter Weise über die Begrenzungseinheit ausgebildet sein. Diese kann insbesondere mit einer oder mehreren radial positionierten elastischen Lamellen ausgestattet sein, die einerseits verdrehsicher mit der Spindel verbunden sind und andererseits in eine in dem Griff ausgebildete Rastkontur eingreifen.

Grundsätzlich ist diese Lamellenkonstruktion auch abwandelbar, solange die Drehbewegung des Drehhandgriffs über einen Reib- oder elastischen Formschluss auf die Spindel übertragen wird, wobei der Reibschluss oder der Formschluss das maximal übertragbare Drehmoment bestimmt. Beispielsweise können die Lamellen anstelle deren radialer Ausrichtung auch axial ausgerichtet sein und sich hierfür beispielsweise an einer freien axialen Stirnfläche des Drehhandgriffs oder der Spindel anordnen sowie in eine entsprechend axial angeordnete Rastkontur am gegenüberliegenden Bauteil eingreifen. Auch ist es nicht unbedingt erforderlich die Lamellen federelastisch auszubilden. Vielmehr könnten die Lamellen auch starr ausgeführt und stattdessen deren Lagerung federelastisch sein.

Eine weitere Ausführungsform der Erfindung ist dadurch gekennzeichnet, dass die Spindel ein einseitig offenes Spindelgewinde aufweist. Dieses kann im Rahmen der Erfindung in Schließrichtung und/oder in Öffnungsrichtung der Spindel offen sein. Auf diese Weise ist die aktive Spindelgewindelänge, über die eine Verstellung der Klemmvorrichtung bewirkt werden kann, begrenzt. Bewegt sich die Spindelmutter auf dem Spindelgewinde infolge zu großer Verstellung über das Ende des Spindelgewindes hinaus, gelangen die Spindelmutter und die Spindel außer Eingriff und es kann durch fortwährendes Verdrehen der Spindel keine Kraft mehr auf die Klemmbacke übertragen werden. Es ist so eine Überlastsicherung ausgebildet, die eine mechanische Zerstörung der Klemmvorrichtung und der Führung sicher vermeidet. Bei einer Ausführungsform ist das Spindelgewinde in die Öffnungsrichtung der Spindel (gegenüber der Klemmrichtung der Spindel) offen. Dadurch wird eine Überlastsicherung geschaffen, die beim Öffnen der Klemmbacken wirkt, insbesondere in einem Fall, in dem die Klemmbacke vollständig geöffnet wurde (die Spindelmutter liegt dann am Gewindeende in Anschlag) und ein Anwender dennoch mit einer Verstellung fortfährt. Da Spindel und Spindelmutter dann außer Gewindeeingriff sind, dreht die Spindel gegenüber der Spindelmutter quasi leer, so dass sich Spindelmutter und Spindel nicht verklemmen können.

Es ist besonders vorteilhaft, wenn endseitig der Spindel ein insbesondere elastisches Anschlagelement angeordnet ist, derart, dass die Spindelmutter oder die Spindel an dem Anschlagelement zur Anlage kommen, sobald die Spindelmutter und die Spindel am offenen Spindelgewindeende außer Eingriff gelangt sind. Das Anschlagelement kann nach der Erfindung in besonders vorteilhafter Weise zwischen dem Spindelgewindeende und einem Gehäuse eines zu befestigenden Medizingeräts angeordnet oder ausgebildet sein. Es ist vorzugsweise derart positioniert, dass die Spindelmutter daran nach, und insbesondere unmittelbar nach, einem Außer-Eingriff-Gelangen vom Spindelgewinde zur Anlage kommt. Infolge der Elastizität des Anschlagelements wird die Spindelmutter nach dem Verlassen des Spindelgewindes zurück in Richtung des Spindelgewindes gedrückt. Solange die Drehrichtung der Spindel beibehalten wird, rutschen Spindelmutter und Spindel gegenseitig durch. Sobald aber die Drehrichtung der Spindel umgekehrt wird, wird die Spindelmutter infolge der durch das elastische Element auf sie ausgeübten Vorspannung in Richtung der Spindel automatisch zurück in Eingriff mit dem Spindelgewinde gebracht. Es ist auf diese Weise sichergestellt, dass die Klemmbacke auch bei einem vollständigen Herausdrehen (außer Eingriff gelangen) oder Lösen von Spindel und Spindelmutter stets wieder ohne Probleme in Klemmrichtung angezogen werden kann.

Nach einer Ausführungsform der Erfindung ist vorgesehen, dass das Anschlagelement aus einem Elastomer, Gummi oder Metall, insbesondere aus einem Federstahl besteht und/oder in Form eines Rings, einer Spiralfeder oder einer Blattfeder ausgebildet ist.

Eine weitere Ausführungsform der Erfindung ist dadurch gekennzeichnet, dass die Begrenzungseinheit eine oder mehrere radial positionierte elastische Lamellen aufweist, die drehfest mit der Spindel verbunden ist bzw. sind und mit ihrem jeweils radial äußeren Ende in eine in dem Drehhandgriff ausgebildete Rastkontur eingreift. Alternativ können die Lamellen am Drehhandgriff, insbesondere an einem Gehäuse des Drehhandgriffs, drehfest angeordnet sein und in an der Spindel ausgebildete Rastkonturen eingreifen. Die Lamellen sind vorzugsweise so gestaltet, dass sie bei Überschreiten einer Grenzdrehkraft bei einer Relativbewegung zwischen der Spindel und dem Drehhandgriff in eine erste Richtung an der Rastkontur ausweichen oder abgleiten können. Bei einer Relativbewegung in die entgegengesetzte Richtung bewirken sie ein Sperren durch einen Eingriff mit der Rastkontur. Auf diese Weise kann zum Öffnen der Klemmbacke ein größeres Moment übertragen werden als zum Schließen. Die Rastkontur ist vorzugsweise durch in radialer Richtung ausgebildete Nuten/Vertiefungen und/oder Vorsprünge ausgebildet.

Der Grenzwert, bei dessen Überschreiten es zu einem Durchrutschen der Begrenzungseinheit kommt, ist durch bestimmte Eigenschaften definiert. Zu nennen sind in diesem Zusammenhang insbesondere die Festigkeit (Steifigkeit) des Materials des Kupplungselements und/oder die Anzahl der Lamellen und/oder die Tiefe der Rastkontur und/oder der Außendurchmesser der Lamellen und/oder die Wandstärke der Lamellen und/oder die Breite der Lamellen. Die vorgenannten Eigenschaften bestimmen die Neigung der Lamellen, bei Kontakt mit der Rastkontur dieser auszuweichen. Durch geeignete Auswahl solcher Eigenschaften kann eine entsprechende nominale Drehmomentbegrenzung erzielt werden, infolge deren eine entsprechende Normalkraft über die Spindel und die Spindelmutter auf die Klemmbacke übertragen wird und somit eine entsprechende Haltekraft des Gesamtbefestigung erzielt wird.

Die Lamellen der Rutschkupplung können aus einem metallischen Werkstoff, insbesondere aus Federstahl, und/oder aus einem Kunststoff, insbesondere aus PP, PE-HD, PE-LD, POM, ABS oder einem ähnlichen technischen Kunststoff oder Kombinationen davon bestehen.

Die Abstände der nach innen gerichteten Lamellen voneinander sind vorzugsweise so ausgebildet, dass ein Anwender bei einem Durchrutschen der Begrenzungseinheit ein Rastgeräusch oder eine Reihe von Rastgeräuschen wahrnimmt. Auf diese Weise wird dem Anmelder akustisch oder auch haptisch vermittelt, dass er den Griff über die Drehmomentbegrenzung hinaus zum Beispiel um noch eine halbe Umdrehung weiter betätigt. Die Geometrie und die Oberflächenbeschaffenheit der Lamellen und/oder der Rastkontur kann bzw. können nach der Erfindung derart ausgebildet sein, dass die Lamellen beim Überdrehen sanft abgleiten. Dabei werden die Gleitflächen der Lamellen und der Rastkontur nicht oder nur unwesentlich beansprucht, um eine dauerhaft gleichbleibende Drehmomentbegrenzung zu erzielen.

Die Klemmbacken können jeweils eine Anlagestruktur zum Zusammenwirken mit einer Führung aufweisen. Die Anlagestruktur bildet eine Auflage aus, mit der die Klemmbacke die Führung kontaktiert. Sie ist vorzugsweise mit speziellen Materialeigenschaften ausgebildet und kann insbesondere aus einer Kombination einer Hartkomponente mit einer Weichkomponente mit definiertem Reibkoeffizienten und definierter Härte besteht, um bei angezogener Klemmbacke dauerhaft ein Abgleiten oder ein Verdrehen des Medizingerätes von oder an der Führung zu vermeiden. Die Anlagestruktur kann insbesondere chemikalienbeständig sein, um bei angezogener Klemmbacke ein Abgleiten oder ein Verdrehen des Medizingerätes an der Stange dauerhaft vermeiden zu können. Sie kann insbesondere austauschbar ausgebildet sein, so dass im Falle von Alterung, Abnutzung oder Zerstörung ein Ersetzen problemlos möglich ist.

Eine besonders sichere Befestigung kann erzielt werden, wenn zumindest eine der Klemmbacken, vorzugsweise beide oder alle Klemmbacken mit einer Vertiefung oder Sicke ausgebildet ist bzw. sind. Diese Vertiefung oder Sicke ist vorzugsweise auf die Form der Führung abgestimmt und stellt sicher, dass z.B. im Falle einer Führung mit kreisförmigem Querschnitt unterschiedliche Querschnittsdurchmesser in eine Richtung so zentriert werden, dass das Medizingerät stets parallel zur Führung fixiert ist, nicht schräg montiert wird und die Krafteinleitung stets durch die (Mittel-)Achse der Führung führt. Bei geeigneter Geometrie der Anlagestruktur kann auch bei entfernter Weichkomponente gewährleistet sein, dass eine sichere Befestigung an der Führung möglich ist. Insbesondere kann die Anlagestruktur derart ausgebildet sein, dass auch bei entfernter Weichkomponente eine Dichtigkeit des Gehäuses, beispielsweise IPX4, gewährleistet ist.

Durch die Erfindung können insbesondere die folgenden Vorteile erzielt werden:
- eine Dreh- und somit Kraftbegrenzung durch Verwendung der Begrenzungseinheit wendet die Gefahr einer Zerstörung der Klemmvorrichtung, der Führung sowie des befestigten medizintechnischen Geräts ab;
- ein mechanischer Freilauf der Spindelmutter wendet Gefahr eines Festsitzens beim Lösen der Klemmvorrichtung ab; und
- es werden Auflagen mit einer Geometrie genutzt, welche ein falsches Montieren unterbinden.

Zusammenfassend kann man auch sagen, dass die Erfindung mittels einer in einen Betätigungsgriff integrierten Rutschkupplung das von der Klemmvorrichtung auf die Führung ausgeübte Drehmoment begrenzt. Alternativ oder zusätzlich kann mittels der Rutschkupplung das zum Lösen der Klemmvorrichtung ausgeübte Drehmoment begrenzt werden. Alternativ oder zusätzlich kann durch Integration eines elastischen Elements in Verbindung mit einer definierten Spindellänge mit Freilauf ein Festklemmen der Spindel vermieden werden. Eine Auflage der Klemmbacken, die in Form, Härte und Material auf die mit den Klemmbacken zusammenwirkende Führung abgestimmt ist, kann zur Vermeidung von Fehlmontagen und/oder Abgleiten im Rahmen der Erfindung verwendet werden.

Weitere Merkmale und Vorteile der vorliegenden Erfindung ergeben sich aus der folgenden beispielhaften und nicht beschränkenden Beschreibung der Erfindung anhand von Figuren. Diese sind lediglich schematischer Natur und dienen nur dem Verständnis der Erfindung. Dabei zeigen:
Fig. 1 eine Ausführungsform der Klemmvorrichtung nach der Erfindung in einer perspektivischen Ansicht ohne Gehäuse,
Fig. 2 eine Schnittansicht der Klemmvorrichtung der Figur 1 entlang der Linie II-II und
Fig. 3 eine Ausführungsform der Klemmvorrichtung mit Gehäuse in einer perspektivischen Ansicht.

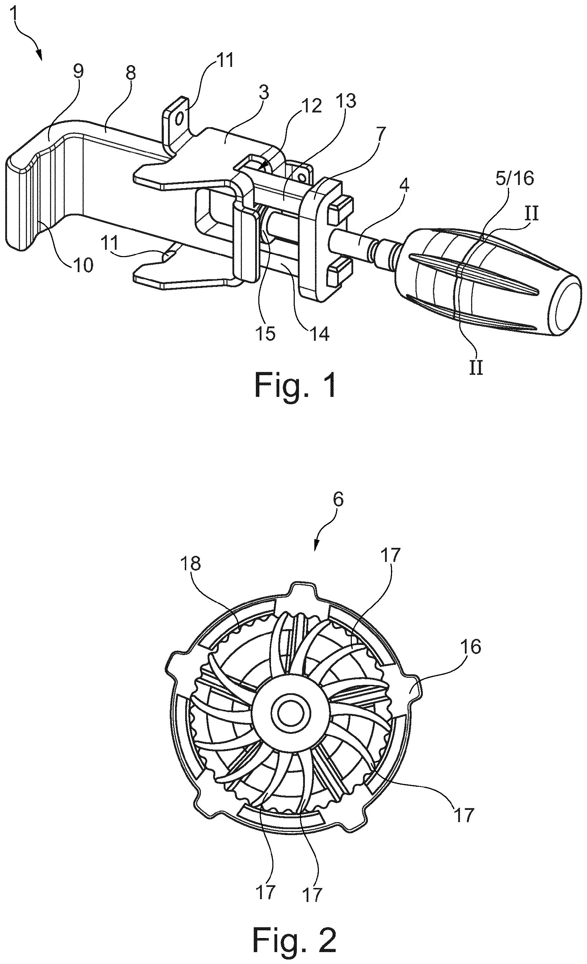

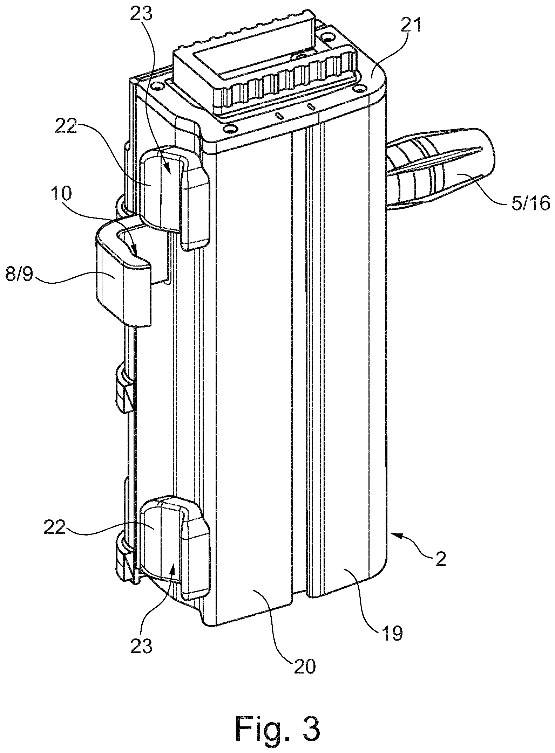

Figur 1 zeigt eine Ausführungsform einer Klemmvorrichtung 1 nach der Erfindung, die ohne Gehäuse 2 dargestellt ist oder aber kein Gehäuse 2 aufweist. Eine Ausführungsform der Erfindung mit Gehäuse 2 ist in Figur 3 gezeigt.

Wie am besten in Figur 1 zu erkennen ist, weist die Klemmvorrichtung 1 eine mittels einer Lagereinheit 3 in axialer Richtung und in radialer Richtung gelagerte, um ihre Längsachse rotierbare Spindel 4 auf. Diese ist mit einem Spindelgewinde mit einer definierten Spindelgewindelänge versehen. Sie ist endseitig, an ihrem proximalen Ende, mit einem Betätigungshandgriff 5 oder einem Knauf oder einer Kurbel versehen, der in Figur 1 auf der rechten Seite gezeigt ist. Der Betätigungshandgriff 5 ist mittels einer im nachfolgenden beschriebenen Begrenzungseinheit 6 mit der Spindel 4 gekoppelt. Bei der mit Gehäuse 2 versehenen Version führt die Spindel 4 auf einer Seite (in Figur 1 auf der rechten Seite) gedichtet aus dem Gehäuse 2 hinaus.

Die Spindel 4 steht über ihr Spindelgewinde mit einer Spindelmutter 7 in Eingriff. Diese ist ebenfalls innerhalb des Gehäuses 2 angeordnet. Die Spindelmutter 7 ist mit einer Klemmbacke 8 wirkverbunden. Alternativ kann sie Bestandteil der Klemmbacke 8 sein.

Die Klemmbacke 8 ragt auf der dem Betätigungsgriff 5 gegenüberliegenden Seite durch eine gedichtete Öffnung aus dem Gehäuse 2 heraus. Das von der Spindelmutter 7 entfernte Ende der Klemmbacke 8 ist als winkliges, hier rechtwinkliges Klemmprofil mit einer Anlagestruktur 9 ausgebildet. In den abgewinkelten Arm der Klemmbacke 8, der senkrecht zur Längsachse der Spindel 4 ausgerichtet ist, ist zu diesem Zweck eine Sicke 10 eingebracht. In einer alternativen Ausführungsform können mehrere Sicken 10 eingebracht sein.

Die Lagereinheit 3 weist zwei Befestigungslaschen 11 auf, mit denen das Gehäuse 2 an der Lagereinheit 3 befestigt wird/ist. Sie weist des Weiteren einen zentralen Durchlass 12 auf, der vom proximalen Ende der Klemmbacke 8 sowie der Spindel 4 durchgriffen ist. Das proximale Ende der Klemmbacke 8 weist zu diesem Zweck zwei proximale parallele Klemmbackenarme 13, 14 auf, zwischen denen ein Freiraum gebildet ist, in den die Spindel 4 eingreift. Das distale Ende der Spindel 4 greift in ein elastisches Anschlagelement 15 ein.

Das Anschlagelement 15 ist derart ausgebildet und positioniert, dass die Spindelmutter 7 beim Öffnen der Klemmvorrichtung 1 in der maximalen Endlage, also im maximal herausgeschraubten Zustand, gegen das Anschlagelement 15 zur Anlage kommt und dieses elastisch verformt. Infolge seiner elastischen Rückstellkraft bei einer solchen Verformung übt das Anschlagelement 15 eine Rückstellkraft auf die Spindelmutter 7 aus, die diese zurück in Richtung der Spindel drängt. Deren Spindelgewinde ist auf der dem Anschlagelement 15 zugewandten Seite der Spindel 4 offen und mit einem in den Figuren nicht dargestellten Freilaufbereich ausgebildet. Die Länge des Freilaufbereichs in Achsrichtung der Spindel entspricht in etwa der Länge der Spindelmutter 7 in Achsrichtung. In der Folge befinden sich die Spindelmutter 7 und das Spindelgewinde nicht in gegenseitigem Eingriff, wenn die Spindelmutter bei Öffnen der Klemmvorrichtung 1 vollständig in den Freilauf eingelaufen ist. Zu diesen Zeitpunkt bzw. in dieser Position ist das Anschlagelement 15 durch die daran anliegende Spindelmutter 7 derart verformt, dass es auf diese eine ausreichend hohe Rückstellkraft ausübt, die die Spindelmutter 7 zurück in Richtung der Spindel 4 und in Gewindeeingriff mit dem Spindelgewinde drängt. Es sei darauf hingewiesen, dass nach der Erfindung ein solcher Gewindefreilauf und ein solches Anschlagelement nicht nur, wie im gezeigten Ausführungsbeispiel, auf der distalen Seite der Spindel ausgebildet sein kann, sondern alternativ auf deren proximalen Seite oder beidseitig, distal und proximal.

Die Begrenzungseinheit 6 ist, wie gut in Figur 2 zu erkennen ist, innerhalb des ein Griffgehäuse 16 aufweisenden Betätigungsgriffs 5 angeordnet. Sie weist eine Mehrzahl von drehfest mit der Spindel 4 verbundenen Lamellen 17 auf. Diese kontaktieren mit ihrem jeweils radial äußeren Ende die radial innere Seite des Griffgehäuses 16. In dieser ist eine Mehrzahl an Rastkonturen 18 ausgebildet, hier in Form von sich in axialer Richtung erstreckenden Nuten, zwischen denen jeweils eine Erhebung liegt. Die Lamellen 17 bilden zusammen mit den Rastkonturen 18 eine Rutschkupplung aus, die als Begrenzungseinheit 6 wirkt. Ein von einem Nutzer beim Festklemmen oder Lösen der Klemmvorrichtung durch eine Drehbetätigung auf den Betätigungshandgriff 5 ausgeübtes Drehmoment wird über die Rastkonturen 18 auf die Lamellen 17 und von diesen auf die Spindel 4 übertragen. Die Lamellen 17 weisen nun eine auf ein Grenzdrehmoment abgestimmte Elastizität auf. Bei Überschreiten dieses Grenzwerts verformen sich die Lamellen 17 derart, dass sie sich aus den Nuten der Rastkontur 18 lösen und auf dieser abgleiten. Die Rutschkupplung rutscht durch. In der dargestellten Ausführungsform rutscht die Begrenzungseinheit 6 nur in eine Drehrichtung durch, nämlich entgegen dem Uhrzeigersinn. Nach der Erfindung kann ein Durchrutschen alternativ in die andere Drehrichtung erfolgen, also in Uhrzeigerrichtung. Es liegt außerdem im Bereich der Erfindung, dass die Begrenzungseinheit 6 in beide Drehrichtungen nach Überschreiten jeweiliger Grenzwerte durchrutschen kann.

Figur 3 zeigt die Klemmvorrichtung 1 mit Gehäuse 2, das mittels der in Figur 1 erkennbaren Befestigungslaschen 11 an der Lagereinheit 3 angeordnet ist. Das Gehäuse 2 umfasst vorliegend zwei Gehäusehälften 19, 20 (diese können auch zu einem Teil vorzugsweise stoffeinstückig zusammengefasst sein) und ist stirnseitig jeweils mit einem Deckel 21 verschlossen. Außenseitig der Gehäusehälfte 20 sind zwei Anlageabschnitte 22 ausgebildet, die distal jeweils mit eine Sicke 23 versehen sind. Diese bilden zusammen mit der Sicke 10 der Klemmbacke 8 die mit einer nicht dargestellten Führung oder Führungsstange zusammenwirkenden Strukturen aus, über die auf die Führung eine Klemmkraft ausgeübt wird und über die die Klemmvorrichtung an der Führung festklemmbar ist.

Die Anlageabschnitte 22 und die Anlagestruktur 9 sind chemikalienbeständig ausgebildet. Auf diese Weise kann bei angezogener Klemmbacke 8 dauerhaft ein Abgleiten oder ein Verdrehen eines mittels der Klemmvorrichtung 1 an der Führung angeordneten Medizingeräts vermieden werden. Sie sind insbesondere austauschbar gestaltet, so dass sie im Falle von Alterung, Abnutzung oder Zerstörung ersetzt werden können. Infolge der in den Figuren dargestellten Geometrie können unterschiedliche Führungsgeometrieen und -durchmesser geklemmt werden. Besonders vorteilhaft ist die in Figur 3 gezeigte Anordnung der Anlageabschnitte 22 und der Anlagestruktur 9, die ein verkantungsfreies Klemmen eines Medizingeräts ermöglicht.

### Bezugszeichenliste

- 1: Klemmvorrichtung
- 2: Gehäuse
- 3: Lagereinheit
- 4: Spindel
- 5: Betätigungshandgriff
- 6: Begrenzungseinheit
- 7: Spindelmutter
- 8: Klemmbacke
- 9: Anlagestruktur, Klemmabschnitt
- 10: Sicke
- 11: Befestigungslasche
- 12: Durchlass
- 13: Klemmbackenarm
- 14: Klemmbackenarm
- 15: Anschlagelement
- 16: Griffgehäuse
- 17: Lamelle
- 18: Rastkontur
- 19: Gehäusehälfte
- 20: Gehäusehälfte
- 21: Deckel
- 22: Anlageabschnitt, Klemmabschnitt
- 23: Sicke

## Patentansprüche

1. Medizintechnische Klemmvorrichtung (1) zur klemmenden Befestigung eines medizintechnischen Geräts an einer Führung, wobei die Klemmvorrichtung (1) zwei Klemmabschnitte (9, 22) für einen klemmenden Eingriff mit der Führung aufweist, von denen zumindest ein Klemmabschnitt (9) relativ zu dem anderen Klemmabschnitt (22) mittels einer Betätigungsmechanik (4, 5, 7) bewegbar ist, wobei
die Klemmvorrichtung (1) eine Begrenzungseinheit (6) aufweist, die kraftabhängig und/oder wegabhängig zumindest den einen Klemmabschnitt (9) von der Betätigungsmechanik (4, 5, 7) entkoppelt,
**dadurch gekennzeichnet, dass**
die Betätigungsmechanik (4, 5, 7) eine mit einer Spindelmutter (7) zusammenwirkende Spindel (4) aufweist, wobei die Spindel (4), welche mit einem Drehhandgriff (5) wirkverbunden und antreibbar ist, axialfest, aber um ihre Spindellängsachse rotierbar in der Klemmvorrichtung (1) gelagert ist, und die Spindelmutter (7) in Richtung der Spindellängsachse axialverschiebbar, aber drehfest in der Klemmvorrichtung (1) gelagert ist, oder die Spindel (4) axial verschiebbar und mechanisch mit einer Klemmbacke nicht rotierbar verbunden ist und die Spindelmutter (7) axial fixiert, jedoch über eine Drehachse rotierbar und mechanisch mit einem Drehhandgriff (5) verbunden ist,
wobei die Klemmvorrichtung (1) ein Gehäuse (2) aufweist, dessen zumindest eine Außenseite einen mit einer durch den Drehhandgriff (5) positionierbaren Klemmbacke (8) zusammenwirkenden Klemmabschnitt (22) ausbildet oder aufweist, und die Klemmbacke (8) und der Klemmabschnitt (22) jeweils eine Anlagestruktur zum Zusammenwirken mit der Führung aufweisen.

2. Medizintechnische Klemmvorrichtung (1) nach Anspruch 1, **dadurch gekennzeichnet, dass** die Begrenzungseinheit (6) in dem Drehhandgriff (5) zur nutzerseitigen Betätigung der Klemmvorrichtung (1) angeordnet ist und vorzugsweise in Form einer Rutschkupplung ausgebildet ist.

3. Medizintechnische Klemmvorrichtung (1) nach einem der vorstehenden Ansprüche, **dadurch gekennzeichnet, dass** die Spindel (4) ein einseitig offenes Spindelgewinde aufweist, das vorzugsweise in die Öffnungsrichtung der Spindel (4) offen ist.

4. Medizintechnische Klemmvorrichtung (1) nach Anspruch 3, **dadurch gekennzeichnet, dass** endseitig der Spindel (4) ein insbesondere elastisches Anschlagelement (15) angeordnet ist, derart, dass die Spindelmutter (7) oder die Spindel (4) an dem Anschlagelement (15) zur Anlage kommen, sobald die Spindelmutter (7) und die Spindel (4) am offenen Spindelgewindeende außer Eingriff gelangt sind.

5. Medizintechnische Klemmvorrichtung (1) nach Anspruch 4, **dadurch gekennzeichnet, dass** das Anschlagelement (15) aus einem Elastomer, Gummi oder Metall, insbesondere aus einem Federstahl besteht und/oder in Form eines Rings, einer Spiralfeder oder einer Blattfeder ausgebildet ist.

6. Medizintechnische Klemmvorrichtung (1) nach einem der Ansprüche 1 bis 5, **dadurch gekennzeichnet, dass** die Begrenzungseinheit (6) eine oder mehrere radial oder axial positionierte elastische oder starre, dafür aber elastisch gelagerte Lamellen (17) aufweist, die drehfest mit der Spindel (4) oder dem Drehhandgriff (5) verbunden ist bzw. sind und mit ihrem jeweils radial oder axial äußeren Ende in eine in dem Drehhandgriff (5) oder der Spindel (4) ausgebildete Rastkontur (18) eingreift.

7. Medizintechnische Klemmvorrichtung (1) nach Anspruch 6, **dadurch gekennzeichnet, dass** die Lamellen (17) aus einem metallischen Werkstoff, insbesondere aus Federstahl, und/oder aus einem Kunststoff, insbesondere aus PP, PE-HD, PE-LD, POM, ABS oder einem ähnlichen technischen Kunststoff oder Kombinationen davon besteht.

8. Medizintechnische Klemmvorrichtung (1) nach einem der vorherigen Ansprüche, mit einer Lagereinheit (3), in welcher die Spindel (4) in axialer Richtung und in radialer Richtung gelagert ist und welche Befestigungslaschen (11) hat, mit denen das Gehäuse (2) an der Lagereinheit (3) befestigt ist.

9. Medizintechnische Klemmvorrichtung (1) nach einem der vorherigen Ansprüche, wobei die Spindel (4) auf einer Seite mit dem Drehhandgriff (5) aus dem Gehäuse (2) herausragt und die Klemmbacke (8) auf der dem Drehhandgriff (5) gegenüberliegenden Seite aus dem Gehäuse (2) herausragt.

10. Medizintechnische Klemmvorrichtung (1) nach einem der vorherigen Ansprüche, wobei ein proximales Ende der Klemmbacke (8) zwei proximale parallele Klemmbackenarme (13, 14) aufweist, zwischen denen ein Freiraum gebildet ist, in den die Spindel (4) eingreift.

## Claims

1. A medical-technical clamping device (1) for clamping attachment of a medical-technical device to a guide, wherein the clamping device (1) has two clamping portions (9, 22) for clamping engagement with the guide, at least one clamping portion (9) of which can be moved relative to the other clamping portion (22) via an actuation mechanism (4, 5, 7), wherein
the clamping device (1) has a limiting unit (6) which decouples at least the one clamping portion (9) from the actuation mechanism (4, 5, 7) in a force-dependent and/or path-dependent manner,
**characterized in that**
the actuation mechanism (4, 5, 7) has a spindle (4) interacting with a spindle groove (7), wherein the spindle (4), which is operatively connected and drivable with a rotational handle (5), is mounted axially fixed but rotatable about its longitudinal axis of the spindle in the clamping device (1), and the spindle nut (7) is mounted in the clamping device (1) so as to be axially displaceable in the direction of the longitudinal axis of the spindle but rotationally fixed, or the spindle (4) is axially displaceable and mechanically connected to a clamping jaw so as not to be rotatable and the spindle nut (7) is axially fixed but rotatable about an axis of rotation and mechanically connected to a rotational handle (5),
wherein the clamping device (1) comprises a housing (2), at least one outer side of which forms or comprises a clamping portion (22) interacting with a clamping jaw (8) positionable by the rotational handle (5), and the clamping jaw (8) and the clamping portion (22) each comprise an abutment structure for interacting with the guide.

2. The medical clamping device (1) according to claim 1, **characterized in that** the limiting unit (6) is arranged in the rotational handle (5) for user-side actuation of the clamping device (1) and is preferably in the form of a friction clutch.

3. The medical-technical clamping device (1) according to one of the preceding claims, **characterized in that** the spindle (4) has a spindle thread which is open on one side and is preferably open in the opening direction of the spindle (4).

4. The medical-technical clamping device (1) according to claim 3, **characterized in that** an in particular elastic stop element (15) is arranged at the end of the spindle (4) in such a way that the spindle groove (7) or the spindle (4) comes to rest against the stop element (15) as soon as the spindle groove (7) and the spindle (4) become disengaged at the open spindle-thread end.

5. The medical-technical clamping device (1) according to claim 4, **characterized in that** the stop element (15) is made of an elastomer, rubber or metal, in particular of a spring steel, and/or is in the form of a ring, a coil spring or a leaf spring.

6. The medical-technical clamping device (1) according to one of the claims 1 to 5, **characterized in that** the limiting unit (6) has one or more radially or axially positioned, elastic or rigid but elastically mounted lamellae (17), which is or are connected in a rotationally fixed manner to the spindle (4) or the rotational handle (5) and engages with its or each radially or axially outer end in a detent contour (18) formed in the rotational handle (5) or in the spindle (4).

7. The medical-technical clamping device (1) according to claim 6, **characterized in that** the lamellae (17) consist of a metallic material, in particular spring steel, and/or of a plastic, in particular PP, PE-HD, PE-LD, POM, ABS or a similar engineering plastic or combinations thereof.

8. The medical-technical clamping device (1) according to one of the previous claims, having a bearing unit (3) in which the spindle (4) is mounted in the axial direction and in the radial direction and which has fastening lugs (11) with which the housing (2) is fastened to the bearing unit (3).

9. The medical-technical clamping device (1) according to one of the preceding claims, wherein the spindle (4) protrudes from the housing (2) on one side with the rotational handle (5) and the clamping jaw (8) protrudes from the housing (2) on the side opposite the rotational handle (5).

10. The medical-technical clamping device (1) according to one of the preceding claims, wherein a proximal end of the clamping jaw (8) comprises two proximal parallel clamping-jaw arms (13, 14) between which a clearance is formed in which the spindle (4) engages.

## Revendications

1. Dispositif de serrage (1) médical pour la fixation par serrage d'un appareil médical à un guidage, dans lequel le dispositif de serrage (1) présente deux sections de serrage (9, 22) pour une prise de serrage avec le guidage, dont au moins une section de serrage (9) est mobile par rapport à l'autre section de serrage (22) au moyen d'une mécanique d'actionnement (4, 5, 7), dans lequel
le dispositif de serrage (1) présente une unité de limitation (6) qui découple en fonction de la force et/ou en fonction de la course au moins l'une section de serrage (9) de la mécanique d'actionnement (4, 5, 7),
**caractérisé en ce que**
la mécanique d'actionnement (4, 5, 7) présente une broche (4) coagissant avec un écrou de broche (7), dans lequel la broche (4) qui est reliée activement et entraînable à une poignée tournante (5), est logée de manière fixe axialement mais rotative autour de son axe longitudinal de broche dans le dispositif de serrage (1), et l'écrou de broche (7) est logé de manière coulissante axialement en direction de l'axe longitudinal de broche mais sans pouvoir tourner dans le dispositif de serrage (1), ou la broche (4) est reliée de manière coulissante axialement et non rotative mécaniquement à une mâchoire de serrage et fixe axialement l'écrou de broche (7), toutefois est reliée de manière rotative et mécanique par le biais d'un axe de rotation à une poignée tournante (5),
dans lequel le dispositif de serrage (1) présente un boîtier (2), dont au moins un côté extérieur réalise ou présente une section de serrage (22) coagissant avec une mâchoire de serrage (8) positionnable par la poignée tournante (5), et la mâchoire de serrage (8) et la section de serrage (22) présentent respectivement une structure d'appui pour la coopération avec le guidage.

2. Dispositif de serrage (1) médical selon la revendication 1, **caractérisé en ce que** l'unité de limitation (6) est agencée dans la poignée tournante (5) pour l'actionnement côté utilisateur du dispositif de serrage (1) et est de préférence réalisée sous la forme d'un couplage glissant.

3. Dispositif de serrage (1) médical selon l'une quelconque des revendications précédentes, **caractérisé en ce que** la broche (4) présente un filet de broche ouvert d'un côté qui est de préférence ouvert dans le sens d'ouverture de la broche (4).

4. Dispositif de serrage (1) médical selon la revendication 3, **caractérisé en ce que** un élément de butée (15) en particulier élastique est agencé côté extrémité de la broche (4) de telle manière que l'écrou de broche (7) ou la broche (4) vienne en appui contre l'élément de butée (15) dès que l'écrou de broche (7) et la broche (4) sont parvenus hors prise au niveau de l'extrémité de filet de broche ouverte.

5. Dispositif de serrage (1) médical selon la revendication 4, **caractérisé en ce que** l'élément de butée (15) se compose d'un élastomère, de caoutchouc ou de métal, en particulier d'un acier sur ressorts et/ou est réalisé sous la forme d'un anneau, d'un ressort hélicoïdal ou d'un ressort à lames.

6. Dispositif de serrage (1) médical selon l'une quelconque des revendications 1 à 5, **caractérisé en ce que** l'unité de limitation (6) présente une ou plusieurs lamelles (17) positionnées radialement ou axialement, élastiques ou rigides, logées à cet effet mais élastiquement, qui est ou sont reliée(s) sans pouvoir tourner à la broche (4) ou la poignée tournante (5) et vient en prise avec son extrémité extérieure respectivement radialement ou axialement dans un contour d'encliquetage (18) réalisé dans la poignée tournante (5) ou la broche (4).

7. Dispositif de serrage (1) médical selon la revendication 6, **caractérisé en ce que** les lamelles (17) se composent d'un matériau métallique, en particulier d'acier sur ressorts, et/ou d'une matière plastique, en particulier de PP, PE-HD, PE-LD, POM, ABS ou d'une matière plastique technique similaire ou de combinaisons de ceux-ci.

8. Dispositif de serrage (1) médical selon l'une quelconque des revendications précédentes, avec une unité de palier (3), dans laquelle la broche (4) est logée dans le sens axial et dans le sens radial et qui présente des languettes de fixation (11), avec lesquelles le boîtier (2) est fixé à l'unité de palier (3).

9. Dispositif de serrage (1) médical selon l'une quelconque des revendications précédentes, dans lequel la broche (4) dépasse du boîtier (2) sur un côté avec la poignée tournante (5) et la mâchoire de serrage (8) dépasse du boîtier (2) sur le côté opposé à la poignée tournante (5).

10. Dispositif de serrage (1) médical selon l'une quelconque des revendications précédentes, dans lequel une extrémité proximale de la mâchoire de serrage (8) présente deux bras de mâchoire de serrage (13, 14) parallèles proximaux, entre lesquels un espace libre est formé, dans lequel la broche (4) vient en prise.
